# FASCICULE DE BREVET EUROPEEN

(11) **EP 0 448 891 B1**
(45) Date de publication et mention de la délivrance du brevet: **01.03.1995**
(21) Numéro de dépôt: 90402717.4
(22) Date de dépôt: 02.10.1990
(51) Int. Cl.: A61F 2/02

(54) **Dispositif destiné à être implanté dans un vaisseau avec des pattes latérales à dents antagonistes**
Blutgefässimplantat mit entgegengesetzten, Widerhaken aufweisenden Faltarmen
Blood vessel implant having legs with opposing points

(30) Priorité: 28.03.1990 FR 9004552
(43) Date de publication de la demande: 02.10.1991
(73) Titulaire: LEFEBVRE, Jean-Marie, F-59800 Lille (FR)
(72) Inventeur: LEFEBVRE, Jean-Marie, F-59800 Lille (FR)
(74) Mandataire: Bruin, Cornelis Willem

(56) Documents cités:
- EP-A- 0 348 295
- DE-A- 3 719 705
- FR-A- 2 573 646
- FR-A- 2 608 418

## Description

La présente invention concerne tous les dispositifs qui sont destinés à être implantés de manière définitive dans un vaisseau et qui comportent des pattes latérales prenant appui sur la paroi interne dudit vaisseau, munies de moyen d'accrochage et dont l'extrémité libre n'est pas effilée; Un tel dispositif est connu du document US-A-3 334 629. La présente invention concerne notamment les filtres que l'on introduit par voie endoveineuse pour empêcher la migration de caillots sanguins. Plus précisément la présente invention concerne les moyens d'accrochage dont sont pourvues les pattes et qui assurent l'ancrage du dispositif dans la paroi du vaisseau.

Dans les dispositifs connus, les moyens d'accrochage ont des formes très diverses , qui sont fonctions de la configuration des pattes. Il s'agit dans les documents FR.A.2.570.288 et US.A.3,952,747 de l'extrémité effilée de chaque patte qui est repliée pour former un crochet. Il s'agit dans le document US.A.3,334,629 d'une multiplicité de petites dents dépassant de la surface externe de l'extrémité libre de chaque patte.

En ce qui concerne les dents d'accrochage en général le document DE-A-3 719 705 décrit un dispositif destiné à être implanté dans une zone de rétrécissement d'un conduit corporel, du type canal biliaire. Ce dispositif, qui est de forme tubulairé, comporte sur sa périphérie des excroissances antagonistes qui ont pour fonction de conférer au dispositif de forme tubulaire, un diamètre suffisamment important pour que le dispositif ne puisse pas passer par la zone de rétrécissement, une fois qu'il est mis en place, en sorte d'assurer son ancrage en position.

Le but visé, pour ces moyens d'accrochage , est de maintenir le dispositif à l'emplacement où il a été implanté dans le vaisseau, en évitant tout déplacement et toute migration du dispositif , mais aussi en évitant de perforer ou endommager la paroi du vaisseau.

Ce but est , selon le demandeur, imparfaitement atteint dans les dispositifs connus. Il résulte des observations qu'il a pu faire qu'étant conçus pour s'opposer à tout déplacement du dispositif dans le sens de l'écoulement du flux à l'intérieur du vaisseau , les moyens d'accrochage précités ne sont pas réellement en mesure d'assurer un ancrage définitif et absolu. En effet la paroi du vaisseau n'est pas un élément rigide ; elle est soumise à des mouvements fluctuants lors du passage du flux , en particulier du flux sanguin dans une vaine. Ces mouvements fluctuants ont une action sur les pattes du dispositif et peuvent entraîner le décrochage de tout ou partie de ces pattes dont les crochets ou dents ont tous la même orientation, avec corrélativement un déplacement du dispositif complet ou un déplacement relatif d'une ou plusieurs pattes par rapport aux autres et donc un mauvais positionnement du dispositif par rapport au vaisseau. Ces mouvements fluctuants peuvent aussi occasionner , en combinaison avec la force d'entraînement du flux , la perforation de la paroi lorsque les moyens d'accrochage sont trop pénétrants.

Le but ci-dessus est parfaitement atteint par le dispositif de l'invention. Il s'agit d'un dispositif destiné à être implanté définitivement dans un vaisseau , par exemple un filtre pour la retenue de caillots sanguins, et qui comporte des pattes latérales qui sont munies de moyens d'accrochage et dont l'extrémité n'est pas effilée. Selon l'invention les moyens d'accrochage consistent en au moins deux dents dirigées , selon la direction générale de la patte, de manière antagoniste, qui sont à proximité l'une de l'autre et qui ont des directions divergentes.

Le terme dent doit être pris ici dans un sens général, comme étant une tige dépassant de la surface de la patte, dont une extrémité est solidaire de la patte et dont l'autre extrémité est pointue.

Ainsi chaque patte comporte deux dents qui sont inclinées par rapport à la surface de la patte , l'une dans le sens de l'écoulement du flux et l'autre dans le sens inverse. Grâce à cette disposition particulière, les mouvements fluctuants de la paroi provoquent l'accrochage dans la paroi des deux dents. Une fois cet accrochage réalisé, il s'oppose à tout mouvement de la patte, dans un sens comme dans l'autre ; l'ancrage obtenu est réellement définitif et absolu. Du fait que l'extrémité des pattes n'est pas effilée , il n'y a pas de risque de pénétration directe des pattes dans la paroi.

Lorsque la partie de la patte qui prend appui sur la paroi du vaisseau est d'une longueur réduite, d'un à quelques centimètres, il est nécessaire que les deux dents soient à proximité l'une de l'autre et, pour obtenir l'effet recherché, leurs directions sont divergentes. Dans le cas contraire, l'intervalle entre les extrémités pointues serait trop petit pour que la paroi du vaisseau puisse s'y introduire lors de l'ouverture du dispositif et l'application des pattes sur la paroi.

Pour éviter les risques de perforations de la paroi, tout en assurant un ancrage efficace, il est préférable que la structure de la dent par rapport à la patte soit telle qu'au moins l'extrémité libre de la dent a une inclinaison par rapport à la patte d'au plus 30 °, la pointe de la dent étant à une distance de la patte comprise entre 0,8 et 2mm. Ainsi la dent ne peut pénétrer dans la paroi que sur une épaisseur réduite.

Ceci peut être obtenu avec une dent rectiligne ; cependant pour limiter la longueur de la dent, il est avantageux que la dent soit recourbée ou pliée , son extrémité solidaire de la patte ayant une inclinaison supérieure à 30 °.

Dans le mode préféré de réalisation, chaque patte du dispositif étant une tige plate en métal, les dents sont obtenues par découpe et repoussage dans la partie médiane proche de l'extrémité libre de la tige.

De préférence chaque patte est munie de deux dents ayant la forme de deux triangles dont les bases parallèles , solidaires de la patte, ont entre elles un écartement compris entre 2 et 10 mm.

D'autres avantages et caractéristiques de l'invention apparaîtront plus clairement à la lecture de la description qui va être faite d'un exemple de réalisation d'un filtre médical pour la retenue de caillots sanguins dont chaque patte latérale est munie de deux dents antagonistes, illustré par le dessin annexé dans lequel :
La figure 1 est une vue de côté d'un filtre,
La figure 2 est une vue de dessus de l'extrémité d'une patte,
La figure 3 est une vue en coupe longitudinale de l'extrémité d'une patte accrochée sur une paroi veineuse.

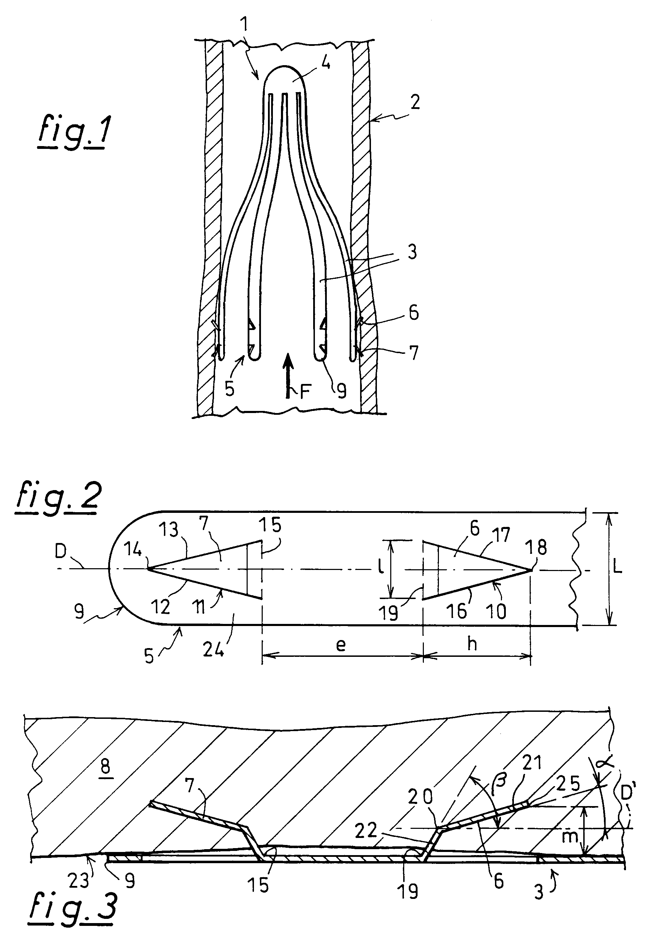

Le filtre 1 est représenté sur la figure 1 alors qu'il est placé à l'intérieur d'une veine 2 qui est parcourue par le flux sanguin dans le sens de la flèche F. Le filtre 1 a une forme générale conique avec les pattes latérales 3 qui sont réunies pour former la tête ogivale 4 du filtre 1. Les extrémités libres 5 de chaque patte latérale 3 ont un bout 9 arrondi et comportent deux dents 6,7.

De manière classique, le filtre est réalisé dans un matériau qui présente une certaine élasticité, de sorte que les pattes 3 peuvent être ramenées sensiblement les unes contre les autres dans une gaine d'introduction du type cathéter et qu'elles se déploient à l'intérieur de la veine 2, lorsque le filtre est poussé hors de la gaine. La déformation élastique des pattes 3 par rapport à la tête ogivale 4 est telle que , le filtre 1 étant implanté, l'extrémité libre 5 de chaque patte latérale 3 prend appui sur la paroi interne 8 de la veine 2.

Selon le mode préféré de réalisation, le filtre 1 est une pièce métallique avec six tiges plates faisant office de pattes 3. Chaque patte 3 a une largeur de l'ordre de 2mm.

On a représenté sur la figure 2 l'extrémité libre 5 d'une patte 3, avec son bout arrondi 9. Pour réaliser les dents 6,7, on effectue deux découpes suivant les traits pleins 10,11. La découpe 11 correspondant à la dent 7, située à proximité immédiate du bout 9, est formée de deux entailles rectilignes 12,13 se coupant en un point 14 appartenant à l'axe de symétrie D de la patte 3. Le point 14 est situé vers le bord arrondi 9. On comprend en examinant la figure 2 que la dent 7 est obtenue en repoussant vers l'extérieur de la patte 3 la partie triangulaire délimitée par les entailles 12,13. La ligne en pointillé 15 constitue l'axe de pliage et donc la base de la dent 7, rendant celle-ci solidaire de la patte 3.

Les entailles 16,17 correspondant à la seconde découpe 10 sont effectuées de la même manière que ci-dessus , exception faite que le point de convergence 18 des deux entailles 16,17 est tourné vers la pointe ogivale 4.

Dans un exemple précis de réalisation, l'extrémité libre 5 de la patte 3 ayant une largeur L de 2mm, les bases 15 et 19 des deux dents avaient une longueur 1 de 1mm ; elles étaient parallèles et écartées l'une de l'autre d'une distance e de 2 ,5mm. La hauteur h du triangle correspondant à la hauteur théorique de la dent était de 1,8mm.

Chaque dent 6,7 est pliée , conformément à la représentation de la figure 3, selon un axe de pliage 20 qui sépare d'un côté la partie extrême et triangulaire 21 de la dent et de l'autre côté la partie 22 attenant à la patte 3 formant un trapèze isocèle dont la grande base est la première ligne 19 selon laquelle la partie interne aux deux entailles 16,17 a été repoussée.

L'angle α formé entre la partie extrême triangulaire 21 de la dent et la direction D'duplan de la patte 3 est de préférence compris entre 0° et 30° ; dans l'exemple précité il était de 15°.

L'angle βformé entre la partie trapézoîdale 22 de la dent et la directionD'du plan de la patte 3 est de préférence compris entre 45 et 90° ; dans l'exemple précité il était de 60°.

Le point 25 de la dent 6 est distant d'une hauteur m de la patte 3, qui est de préférence comprise entre 0,8 et 2mm. Dans l'exemple précité, elle était de 1mm.

Lors de la mise en place du filtre dans la veine 2, les pattes 3 se déploient et leurs extrémités 5 viennent s'appliquer sur la face interne 23 de la paroi 8. Sous la force d'entraînement du flux sanguin, le filtre 1 a tendance à se déplacer dans le sens de la flèche F, ce qui provoque la pénétration de la dent 6 tournée vers la tête ogivale 4 dans la paroi veineuse 8. De plus, les mouvements fluctuants de la paroi 8 favorisent la pénétration progressive de la dent 7 tournée vers le bout arrondi 9. La surface 24 de l'extrémité libre 5 de la patte 3 forme une surface d'appui importante qui empêche toute pénétration de la patte 3 elle-même dans la paroi 8.

Ainsi, après que les dents 6,7 de toutes les pattes 3 ont pris leur place dans la paroi 8 de la veine 2, le filtre 1 est définitivement accroché sans aucune possibilité de migration ultérieure et sans risque d'endommagement de la veine 2.

L'invention n'est pas limitée au mode de réalisation qui a été décrit à titre d'exemple non exhaustif , quant bien même il s'agit du mode préféré, mais elle en couvre toutes les variantes dans le cadre des revendications ci-dessous. En particulier le nombre total de dents sur chaque patte n'est plus obligatoirement de deux ; il importe qu'il y ait au moins deux dents antagonistes. Par ailleurs il revient à l'homme du métier de déterminer , en fonction du type de dispositif , filtrant ou de blocage ou d'écartement, en fonction de ses dimensions et des matériaux constitutifs, la configuration et la disposition des dents antagonistes . On comprend qu'un résultat analogue au pliage selon la ligne 20 peut être obtenu en recourbant la dent selon un arc de cercle , de telle manière que la partie la plus extrême de la dent fasse avec la direction du plan de la patte un angle α . De même , le terme patte n'est pas exclusif des branches servant à la filtration ou à l'écartement, il peut aussi s'agir des appendices prévus dans le document FR.A.2.573.646, qui constituent en quelque sorte les extrémités libres qui prennent appui sur la paroi du vaisseau, dans le sens qui a été donné dans le présent texte.

## Revendications

1. Dispositif destiné a être implanté définitivement dans a vaisseau (2) , et comportant des pattes latérales (3), qui sont munies de moyens d'accrochage et dont l'extrémité libre n'est pas effilée, caractérisé en ce que les moyens d'accrochage consistent en au moins deux dents (6, 7) qui sont dirigées selon la direction générale de la patte (3) de manière antagoniste, qui sont à proximité l'une de l'autre et qui ont des directions divergentes.

2. Dispositif selon la revendication 1 caractérisé en ce que l'extrémité libre (21) de la dent (6) a une inclinaison par rapport à la patte d'au plus 30° et en ce que la pointe (25) de la dent (6) est à une distance (m) de la patte comprise entre 0,8 et 2 mm.

3. Dispositif selon la revendication 2 caractérisé en ce que la dent (6) est recourbée ou pliée, son extrémité (22) solidaire de la patte (3) ayant une inclinaison (β) supérieure à 30°.

4. Dispositif selon la revendication 3 caractérisé en ce que l'inclinaison (β) de l'extrémité (22) de la dent (6) solidaire de la patte est comprise entre 45° et 90°.

5. Dispositif selon la revendication 1 caractérisé en ce que chaque patte (3) étant une tige plate en métal, les dents (6,7) sont obtenues par découpe et repoussage dans la partie médiane.

6. Dispositif selon la revendication 1 caractérisé en ce que chaque patte (3) est munie de deux dents (6, 7) ayant la forme de deux triangles dont les bases (15, 19) parallèles, solidaires de la patte (3), ont entre elles un écartement (e) compris entre 2 et 10 mm.

## Claims

1. Device adapted to be implanted definitively in a vessel (2), and comprising lateral legs (3) which are provided with hooking means, and of which the free end is not being sharp, characterised in that the hooking means consist of at least two teeth (6, 7) which are oriented antagonistically in the general direction of the leg (3), and which are in the vicinity of each other and have divergent directions.

2. Device according to Claim 1, characterised in that the free end (21) of the tooth (6) presents an inclination with respect to the leg of at the most 30° and in that the tip (25) of the tooth (6) is at a distance (m) of between 0.8 and 2 mm from the leg.

3. Device according to Claim 2, characterised in that the tooth (6) is curved or bent, its end (22) fast with the leg (3) presenting an inclination (β) greater than 30°.

4. Device according to Claim 3, characterised in that the inclination (β) of the end (22) of the tooth (6) fast with the leg is between 45 and 90°.

5. Device according to claim 1, characterised in that, each leg (3) being a flat metal rod, the teeth (6, 7) are obtained by cutting out and pushing in the median part.

6. Device according to Claim 1, characterised in that each leg (3) is provided with two teeth (6, 7) shaped as two triangles whose parallel bases (15, 19), fast with the leg (3), are spaced apart by a distance (e) of between 2 and 10 mm.

## Patentansprüche

1. Vorrichtung zur definitiven Implantation in einem Gefäß (2), mit seitlichen Armen (3), die mit Verankerungsmitteln versehen sind, und deren freies Ende nicht zugespitzt ist, dadurch gekennzeichnet, daß die Verankerungsmittel aus wenigstens zwei Zähnen (6, 7) bestehen, die in allgemeiner Richtung des Arms (3) einander entgegengerichtet sind, nahe beieinanderliegen und divergierende Richtungen haben.

2. Vorrichtung nach Anspruch 1, dadurch gekennzeichnet, daß das freie Ende (21) des Zahns (6) eine Neigung gegen den Arm von höchstens 30° hat, und daß die Spitze (25) des Zahns (6) einen Abstand (m) vom Arm von zwischen 0,8 und 2 mm hat.

3. Vorrichtung nach Anspruch 2, dadurch gekennzeichnet, daß der Zahn (6) umgebogen oder gefaltet ist, wobei sein mit dem Arm (3) verbundenes Ende eine Neigung (β) von über 30° hat.

4. Vorrichtung nach Anspruch 3, dadurch gekennzeichnet, daß die Neigung (β) des mit dem Arm verbundenen Endes (22) des Zahns (6) zwischen 45° und 90° liegt.

5. Vorrichtung nach Anspruch 1, dadurch gekennzeichnet, daß, wenn jeder Arm (3) eine flache Metallstange ist, die Zähne (6, 7) durch Ausschneiden und Zurückdrücken im mittleren Bereich gebildet werden.

6. Vorrichtung nach Anspruch 1, dadurch gekennzeichnet, daß jeder Arm (3) mit zwei Zähnen (6, 7) versehen ist, die die Form zweier Dreiecke haben, deren mit dem Arm (3) verbundene parallele Basen (15, 19) untereinander einen Abstand (e) von zwischen 2 und 10 mm haben.
